(19) Europäisches Patentamt
European Patent Office
Office européen des brevets

(11) **EP 4 692 362 A1**

(12) **EUROPEAN PATENT APPLICATION**
published in accordance with Art. 153(4) EPC

(43) Date of publication:
**11.02.2026 Bulletin 2026/07**

(21) Application number: **25745328.2**

(22) Date of filing: **22.01.2025**

(51) International Patent Classification (IPC):
*C12P 7/52* (2006.01)    *C12P 7/42* (2006.01)
*C12N 9/88* (2006.01)    *C12N 9/02* (2006.01)

(52) Cooperative Patent Classification (CPC):
**C12N 9/0004; C12N 9/88; C12P 7/42; C12P 7/52**

(86) International application number:
**PCT/KR2025/001247**

(87) International publication number:
**WO 2025/159503 (31.07.2025 Gazette 2025/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.01.2024 KR 20240009485**

(71) Applicant: **LG Chem, Ltd.
Seoul 07336 (KR)**

(72) Inventors:
• **MOON, Sekwon
Daejeon 34122 (KR)**
• **PARK, Jeyun
Daejeon 34122 (KR)**
• **KIM, Jae Hyung
Daejeon 34122 (KR)**

(74) Representative: **Plasseraud IP
104 Rue de Richelieu
CS92104
75080 Paris Cedex 02 (FR)**

(54) **METHOD FOR PRODUCING 3-HYDROXYPROPIONIC ACID**

(57)    The present invention relates to a 2-step method for preparing 3-HP, comprising a first step of culturing cells at a high concentration and a second step of producing 3-HP using the high-concentration cultured cells as a catalyst, which is a method for preparing 3-HP and/or a method for improving productivity of 3-HP by adjusting the DO and/or culture temperature and pH in the second step, while controlling the cell production rate through high-concentration cell culture in the first step, and can produce a high concentration of 3HP from a high concentration of glycerol while stably maintaining cell activity.

【FIG. 1】

EP 4 692 362 A1

**Description**

[TECHNICAL FIELD]

Cross-reference to related applications

The present application claims the benefit of the priority based on Korean Patent Application No. 10-2024-0009485 filed on January 22, 2024, and the entire contents disclosed in the documents of the corresponding Korean patent application are incorporated as a part in the present description.

The present application relates to a method for preparing 3-hydroxypropionic acid (hereinafter, also referred to as "3-HP" ) and/or a method for improving productivity of 3-HP, and more specifically, relates to a method for preparing 3-HP and/or improving productivity of 3-HP, which enable to produce a high concentration of 3-HP from a high concentration of glycerol, while stably maintaining the activity of cells having 3-HP production ability.

[BACKGROUND ART]

3-hydroxypropionic acid is a platform compound that can be converted into various chemical substances such as acrylic acid, methyl acrylate, acrylamide, and the like. Since being selected as a Top 12 value-added biochemical from US Department of Energy (DOE) in 2004, it has been actively studied in the academic world and in the industry.

Production of 3-HP is achieved by largely two methods which are a chemical method and a biological method, but the chemical method has been criticized as it is non-environmentally friendly because initial materials are expensive and toxic substances are produced during the production process, and the like, and therefore, an environmentally friendly bioprocess has been spotlighted.

Glucose and glycerol are mainly used as a substrate for biosynthesis of 3-HP using microorganisms, but due to low yield and productivity, despite of the possibility to produce 3-HP, continuous research on strain development and production process development is still needed.

[DISCLOSURE]

[TECHNICAL PROBLEM]

Accordingly, the present inventors have developed fermentation technology that can increase the 3-HP production concentration while stably maintaining cell activity for the commercialization of 3-HP.

One embodiment of the present application provides a method for preparation of 3-hydroxypropionic acid (3-HP) and/or a method for improving productivity of 3-HP, comprising mass-producing them while controlling the cell production rate through cell culture of cells having productivity of 3-hydroxypropionic acid (3-HP) at a high concentration; and producing 3-HP under the optimal dissolved oxygen (hereinafter, also referred to as "D0" ) and optimal cell stabilization condition and production condition.

[TECHNIAL SOLUTION]

The present application provides a method for preparation of 3-hydroxypropionic acid (3-HP) and/or a method for improving productivity of 3-HP, comprising mass-producing them while controlling the cell production rate through cell culture of cells having productivity of 3-hydroxypropionic acid (3-HP) at a high concentration; and producing 3-HP under the optimal dissolved oxygen and optimal cell stabilization condition and production condition.

More specifically, the method,

comprises (1) high-concentration cell culturing which conducts cell culture of cells having productivity of 3-hydroxypropionic acid (3-HP) in a culture medium (growth medium) until the cell concentration reaches an $OD_{600}$ of 100 to 200; and
(2) producing 3-HP while maintaining the state in which DO (dissolved oxygen) is 1 to 20% by transferring the culture solution of the step (1) to a production medium comprising a substrate,
wherein the step (2) may be performed under the following conditions:

(i) the temperature condition of 30 to 40°C (specifically, 35°C); and/or
(ii) the pH condition of 6 to 8 (specifically, pH 6.8).

Hereinafter, the present invention will be described in more detail.

In the step (1) of the method for preparation of 3-HP and/or method for improving productivity of 3-HP provided in the present description, cells having productivity of 3-hydroxypropionic acid are high-concentration cultured in a culture medium (growth medium). After the high-concentration culture, 3-HP may be produced using the cell culture solution as it is in the step (2) without a separate cell recovery process, specifically, the method may transfer the culture solution itself to a production medium without passing through an additional step including a step of cell recovery, after the culturing of the step (1).

In the step (1), the culture medium (growth medium) may not comprise glycerol as a carbon source.

In the present description, the 3-hydroxypropionic acid producing cell (hereinafter, interchangeably used with the same meaning as 'cell having productivity of 3-hydroxypropionic acid' ) may be selected from microorganisms which can produce 3-HP from a carbon source (for example, glycerol) in a production medium, for example, microorganisms consisting of microorganisms of the genus *Escherichia* (*E. coli,* etc.), the genus Pseudomonas, the genus Enterobacteria, the genus Brevibacterium, the genus Corynebacterium, the genus Klebsiella, the genus Citrobacter, the genus Clostridium, the genus Streptomyces, the genus Bacillus, the genus Lactobacillus, the genus Pseudomonas, the genus Saccharomyces and the genus Aspergillus, but not limited thereto. In one specific embodiment, the 3-hydroxypropionic acid producing cell may be *E. coli.*

In one embodiment, the 3-hydroxypropionic acid producing cell may comprise a gene encoding at least one (for example, 1 or 2 all) selected from the group consisting of glycerol dehydratase and aldehyde dehydrogenase. In one embodiment, the 3-HP producing cell may further comprise a gene (gdrAB) encoding glycerol dehydratase reactivase (GdrAB). In one embodiment, the 3-HP producing cell may be a cell that can biosynthesize vitamin B12 additionally.

The glycerol dehydratase may be encoded by dhaB gene (GenBank accession no. U30903.1), but not limited thereto. The dhaB gene may be an enzyme derived from Klebsiella pneumonia, but not limited thereto. The gene encoding glycerol dehydratase may comprise a gene encoding dhaB1, dhaB2 and/or dhaB3. The glycerol dehydratase protein and a gene encoding thereof may comprise a mutation of the gene and/or amino acid sequence within a range that maintains enzyme activity of degrading glycerol to 3-hydroxypropanal (3-HPal) and water ($H_2O$).

The gene encoding aldehyde dehydrogenase (ALDH) (aldH), may be, for example, aldH (GenBank Accession no. U00096.3; EaldH) gene derived from an Escherichia coli or E. coli K12 MG1655 cell line, puuC gene derived from Klebsiella pneumonia (K. pneumonia), and/or KGSADH gene derived from Azospirillum brasilense, but not limited thereto. The aldehyde dehydrogenase protein and gene encoding thereof may comprise a mutation of the gene and/or amino acid sequence within a range that maintains activity to produce 3-HP from 3-HPal.

The 3-HP producing cell may comprise a gene encoding at least one, at least two, or all the 3 kinds of proteins selected from the group consisting of glycerol dehydratase, aldehyde dehydrogenase, and glycerol dehydratase reactivase, or a recombinant vector comprising the gene.

The recombinant vector may be used by replacing a promoter and a regulatory site within a range of the purpose to express a gene encoding at least one, at least two, or all the 3 kinds of proteins selected from the group consisting of glycerol dehydratase, aldehyde dehydrogenase, and glycerol dehydratase reactivase in a cell by a method known in the art.

The high-concentration culturing may be performed using a method known in the art without limitation within a range of the purpose to secure the 3-HP producing cells in large quantities, and in one embodiment, the culturing may be performed by fed-batch culture.

In one embodiment, the fed-batch culture may be performed by the pH-stat method, DO-stat feeding method, continuous feeding method, or a combination thereof. In one embodiment, when the fed-batch culture using the pH-stat method is performed, glucose may be added at a concentration of 1 to 5g/L.

In one embodiment, when the fed-batch culture using the continuous feeding method is performed, glucose may be added at a rate of 7 to 21 g/L/h.

According to one embodiment, the pH of the culture solution may be adjusted by adding a compound such as ammonium hydroxide, potassium hydroxide, ammonia, phosphate and sulfate into a microorganism culture solution in an appropriate manner during culturing. In one embodiment, during high-concentration culture, the pH may be maintained at 5 to 7.5, 5 to 7, 5.5 to 7.5, 5.5 to 7, 6 to 7.5 or 6.5 to 6, but not limited thereto.

The temperature of the culture solution may be 20°C to 45°C, 25°C to 40°C or 30°C to 37°C, and, it may be, for example, 37°C.

For high-concentration cell culture, the 3-HP producing cell may be produced (proliferated) at a concentration of $OD_{600}$ per hour (optical density (O.D.) measured at 600nm) of 5 to 23, 5 to 22, 5 to 17, 5 to 16, 5 to 10, 5 to 8, 6 to 23, 6 to 22, 6 to 17, 6 to 16, 6 to 10, 6 to 8, 10 to 23, 10 to 22, 10 to 17, 10 to 16, 14 to 23, 14 to 22, 14 to 17, 14 to 16, 17 to 23, 17 to 22, 20 to 23, or 20 to 22, for example, 7, 15, or 21, but not limited thereto, and in the method for preparation of 3-HP and/or method for improving productivity of 3-HP, in the aspect of 3-HP production, it is advantageous that the 3-HP producing cell is produced (proliferated) within the range.

In one embodiment, the cell concentration after the high-concentration culture may be $OD_{600}$ 10 or more, 50 or more, 100 or more, 150 or more, 200 or more, 10 to 500, 10 to 400, 10 to 300, 10 to 250, 50 to 500, 50 to 400, 50 to 300, 50 to 250, 100 to 500, 100 to 400, 100 to 300, 100 to 250, 150 to 500, 150 to 400, 150 to 300, 150 to 250, 200 to 500, 200 to 400, 200 to 300, or 200 to 250, but not limited thereto. Specifically, the cell concentration may be 100 to 200 in $OD_{600}$.

In one embodiment, during the high-concentration culturing, the carbon source comprised in the culture medium (growth medium) may be used by selecting monosaccharides, disaccharides, and/or polysaccharides within a range of the purpose for high-concentration culture without limitation. For example, the carbon source may be at least one, two or more, three or more, four or more, five or more, 10 or more, or all the 11 kinds selected from the group consisting of glucose, fructose, galactose, mannose, arabinose, xylose, ribose, sucrose, maltose, lactose, and cellobiose. The growth medium may not comprise glycerol as the carbon source.

The culture medium (growth medium) may comprise the carbon source at a concentration of 1 to 50 g/L, 1 to 40 g/L, 1 to 30 g/L, 1 to 25 g/L, 1 to 23 g/L, 10 to 50 g/L, 10 to 40 g/L, 10 to 30 g/L, 10 to 25 g/L, 10 to 23 g/L, 15 to 50 g/L, 15 to 40 g/L, 15 to 30 g/L, 15 to 25 g/L, 15 to 23 g/L, 17 to 50 g/L, 17 to 40 g/L, 17 to 30 g/L, 17 to 25 g/L, or 17 to 23 g/L, for example, 20 g/L, based on the volume of the culture medium (growth medium), but not limited thereto.

In order to produce the 3-HP producing cells within the range of $OD_{600}$ (optical density (O.D.) measured at 600nm) per hour, the carbon source may be further supplied at a constant rate.

In one embodiment, in the high-concentration cell culture (the step (1)), the carbon source may be further supplied in the medium at a content of 4 to 25 g/L, 4 to 23 g/L, 4 to 21 g/L, 4 to 17 g/L, 4 to 15 g/L, 4 to 13 g/L, 4 to 9 g/L, 4 to 7 g/L, 7 to 25 g/L, 7 to 23 g/L, 7 to 21 g/L, 7 to 17 g/L, 7 to 15 g/L, 7 to 13 g/L, 7 to 9 g/L, 10 to 25 g/L, 10 to 23 g/L, 10 to 21 g/L, 10 to 17 g/L, 10 to 15 g/L, 13 to 25 g/L, 13 to 23 g/L, 13 to 21 g/L, 13 to 17 g/L, 13 to 15 g/L, 17 to 25 g/L, 17 to 23 g/L, 17 to 21 g/L, 20 to 25 g/L, 20 to 23 g/L, or 20 to 21 g/L, for example, 7, 14, or 21 g/L (concentration; weight of added carbon source (g)/volume of medium (L)) per hour based on the volume of the culture medium (growth medium). The addition of the carbon source in the above range is advantageous for 3-HP production and/or improvement in 3-HP productivity.

The carbon source supplied additionally may be additionally supplied at the time when the concentration of the carbon source in the culture medium (growth medium) is 0 g/L, and specifically, it may be supplied after 5 hours, 6 hours, 7 hours, 8 hours, 9 hours, 10 hours, 11 hours, 12 hours, 13 hours, 14 hours, or 15 hours from the time of the start of cell culture in the culture medium (growth medium), but not limited thereto.

In one example according to the present invention, a glucose solution at a concentration of 700g/L was added to the medium for high-concentration cell culture, and the carbon source solution was supplied to the medium at 10 to 30mL/h per 1L of the medium, so that the sugars added to the culture medium were consumed all and the glucose concentration was maintained at 0g/L.

According to one embodiment, the medium may comprise a nitrogen source and a trace element component, with the carbon source. The available nitrogen source may include peptone, yeast extract, meat juice, malt extract, corn steep liquor, soybean meal and urea or inorganic compounds, for example, ammonium sulfate, ammonium chloride, ammonium phosphate, ammonium carbonate and ammonium nitrate. The nitrogen source may be also used individually or as a mixture, but not limited thereto. The available phosphorus source may include potassium dihydrogen phosphate or dipotassium hydrogen phosphate or sodium-containing salts corresponding thereto, but not limited thereto. In addition, the medium may contain a metal salt such as magnesium sulfate or iron sulfate required for growth, but not limited thereto. In addition, essential growth substances such as amino acids and vitamins may be included. Furthermore, precursors suitable for the medium may be used. The medium or individual component may be added to the culture solution in a fed-batch method or in a continuous method by an appropriate method during the culturing process, but not limited thereto.

The step (2) is a step of transferring the culture solution of the step (1) into a production medium comprising a substrate to produce 3-HP while maintaining the state where DO (dissolved oxygen) is 1 to 20%, and the step (2) is a step performed under the following conditions.

(i) the temperature condition of 30 to 40°C (specifically, 35°C); and/or
(ii) the pH condition of 6 to 8 (specifically, pH 6.8).

Specifically, the step (2) is a step of producing 3-HP under the conditions where dissolved oxygen is maintained at a constant level, and also, the temperature and/or pH are maintained at a constant level for cell stability, by inoculating cells having productivity of 3-hydroxypropionic acid (3-HP) into a 3-hydroxypropionic acid production medium and transferring into the production medium.

The 3-hydroxypropionic acid culture medium (growth medium) may be used without limitation within a range of the purpose of enabling the cells to produce 3-HP without causing proliferation (cell division, growth, or growth and development) of the 3-HP producing cells.

In one embodiment, the carbon source of the culture medium (growth medium) may be glycerol, but not limited thereto. In one embodiment, the medium for producing may further comprise vitamin B12. In one embodiment, the production medium may not comprise glucose during cell inoculation.

In one embodiment, the medium may be synthetic media or semisynthetic media, but not limited thereto.

The cells for inoculation may be prepared in a form of cell culture solution without a separate process of cell recovery after high-concentration culture of cells having productivity of 3-hydroxypropionic acid (3-HP) in the step (1), but not limited thereto.

The inoculation concentration of the high-concentration cultured cells (cell concentration during inoculation) may be appropriately adjusted or determined by those skilled in the art within the range of purpose to producing 3-HP. In one embodiment, the inoculation concentration (based on dry cell weight (DCW)/medium volume (L)) may be 1 to 20 g/L, 1 to 16 g/L, 1 to 12 g/L, 1 to 9 g/L, 2 to 20g/L, 2 to 16 g/L, 2 to 12 g/L, 2 to 9 g/L, 4 to 20 g/L, 4 to 16 g/L, 4 to 12 g/L, or 4 to 9 g/L, but not limited thereto.

In the step of producing 3-hydroxypropionic acid, a culture method known in the art may be selected and used without limitation within a range of purpose to produce 3-HP, and in one embodiment, the step of culturing may be performed by fermentation culture.

In the step of producing 3-HP, proliferation of the inoculated cells may not occur. In one embodiment, the number of the cells at the end of the step of producing may be 150% or less, 130% or less, 100% or less, 90% or less, or 80% or less, for example, 50 to 150%, 50 to 130%, 50 to 100%, 50 to 90%, 50 to 80%, 70 to 150%, 70 to 130%, 70 to 100%, 70 to 90%, or 70 to 80% of the number of the inoculated cells, but not limited thereto.

In one embodiment, the step (2) of the method may maintain the state where dissolved oxygen (DO) is 1 to 20%, 1 to 15%, 1 to 12%, 1 to 10%, 1 to 8%, 1 to 6%, 1 to 5%, 3 to 20%, 3 to 15%, 3 to 12%, 3 to 10%, 3 to 8%, 3 to 6%, 3 to 5%, 4 to 20%, 4 to 15%, 4 to 12%, 4 to 10%, 4 to 8%, 4 to 6%, 4 to 5%, 5 to 20%, 5 to 15%, 5 to 12%, 5 to 10%, 5 to 8%, or 5 to 6%, for example, 5%, but not limited thereto.

In the present description, "change value of dissolved oxygen" may mean a change value of dissolved oxygen during culturing and/or specific substance production processes, and specifically, may be a difference between the maximal value and minimal value of dissolved oxygen during culturing and/or specific substance production processes, and for example, when the minimal value of dissolved oxygen is 4%, and the maximal value is 6% during culturing and/or specific substance production processes, the change value of dissolved oxygen may be expressed as 2%.

The change value of dissolved oxygen in the step (2) of the method may be less than or equal to (less than) 10%, less than or equal to (less than) 7%, less than or equal to (less than) 5%, less than or equal to (less than) 3%, less than or equal to (less than) 2%, less than or equal to (less than) 1%, 0 to 10%, 0 to 7%, 0 to 5%, 0 to 3%, 0 to 2% or 0 to 1%, but not limited thereto.

The maintaining DO (dissolved oxygen) constantly (for example, DO of 4 to 6%), may be performed by at least one selected from the group consisting of stirring rate control, air supply control, and pressure control, and the like during culturing, but not limited thereto. For example, the dissolved oxygen in the medium or culture may be maintained in a certain range, (1) by increasing at least one of the stirring rate, air supply, and pressure, when the dissolved oxygen in the medium or culture is lower than the range, and (2) by reducing at least one of the stirring rate, air supply, and pressure, when the dissolved oxygen in the medium or culture is higher than the range, but not limited thereto. Specifically, in the examples according to the present invention, in order to maintain DO 5%, 3-HP was produced by sequentially adjusting the stirring rate ad air supply during culturing to form an aerobic condition.

In one embodiment, to transfer oxygen accurately, so that the DO condition can be maintained, a process of removing bubbles on the surface of the culture solution may be further comprised. For example, this process of removing bubbles may use an antifoaming agent. As the antifoaming agent, an antifoaming agent that does not affect the cell stability may be used, and an amount at a level at which bubbles are not generated even under the maximal stirring rate and air supply may be added, and this may be appropriately selected by those skilled in the art. For example, using an antifoaming agent such as fatty acid polyglycol ester, bubble generation may be inhibited.

In one embodiment, the method for producing 3-HP by maintaining (adjusting) the dissolved oxygen may increase the 3-HP production amount by 30% or more, 50% or more, 70% or more, 30 to 1000%, 30 to 500%, 30 to 300%, 30 to 100%, 50 to 1000%, 50 to 500%, 50 to 300%, 50 to 100%, 70 to 1000%, 70 to 500%, 70 to 300%, or 70 to 100% compared to a control group not comprising the condition of maintaining dissolved oxygen, but not limited thereto.

In one embodiment, for cell stabilization, the step (2),

may be performed under (i) the temperature condition of 30 to 40°C (specifically, 35°C); and/or
(ii) the pH condition of 6 to 8 (specifically, pH 6.8).

More specifically, the step (2)

(i) may be performed under the temperature condition of 37°C to 40° C , or the pH condition of 7 to 8, and
(ii) may be performed under the temperature condition of 30°C or more to less than 37°C, or the pH condition of 6 or more to less than 7.

More specifically, the step (2)

may be performed under (i) the temperature condition of 35°C; and/or
(ii) the pH condition of 6.8.

According to one embodiment, to adjust the pH in the above range constantly, a compound as selected from the group consisting of calcium hydroxide, magnesium hydroxide, ammonium hydroxide, sodium hydroxide and potassium hydroxide may be added to microorganism culture solution in an appropriate manner during culturing. Additionally, according to one embodiment, to maintain the temperature of the culture solution, a common method for maintaining temperature may be used.

The culturing period may be continued until the desired yield of a useful substance (for example, 3-HP) is obtained, and for example, it may be 3 to 60 hours, 3 to 48 hours, 3 to 36 hours, 3 to 28 hours, 6 to 60 hours, 6 to 48 hours, 6 to 36 hours, 6 to 28 hours, 12 to 60 hours, 12 to 48 hours, 12 to 36 hours, 12 to 28 hours, 20 to 60 hours, 20 to 48 hours, 20 to 36 hours or 20 to 28 hours, and for example, it may be 24 hours, but not limited thereto.

The 3-HP yield of the method for preparation of 3-HP and/or method for improvement of productivity of 3-HP provided in the present description may be for example, 80% or more, 85% or more, 90% or more, 93% or more, or 95% or more, but not limited thereto. The 3-HP yield may be calculated as the 3-HP production amount in the medium (culture) compared to the used amount (moles) of glycerol in the medium (production medium) in the step of producing 3-HP, and in one embodiment, it may be calculated by the following Equation 1.

3-HP production yield (%) = [(Final 3-HP(g))/{( Glycerol before culturing (g)) - (Glycerol remaining after culturing (g))}] * 100      [Equation 1]

The 3-HP productivity showing the 3-HP production amount (production concentration) according to the 3-HP production time of the method for preparation of 3-HP and/or method for improvement of productivity of 3-HP provided in the present description may be calculated by the following Equation 2. Specifically, the 3-HP production amount may be the 3-HP production concentration measured by HPLC measurement and the like.

3-HP productivity = (Final 3-HP production concentration (g/L))/(Total 3-HP production time)      [Equation 2]

The 3-HP productivity of the method for preparation of 3-HP and/or method for improvement of productivity of 3-HP provided in the present description may be 4.0 g/L/h or more, 4.5 g/L/h or more, 4.0 to 60 g/L/h, 4.0 to 40 g/L/h, 4.0 to 20 g/L/h, 4.0 to 10 g/L/h, 5.0 to 60 g/L/h, 5.0 to 40 g/L/h, 5.0 to 20 g/L/h, 5.0 to 10 g/L/h, 6.0 to 60 g/L/h, 6.0 to 40 g/L/h, 6.0 to 20 g/L/h, or 6.0 to 10 g/L/h, but not limited thereto.

Other embodiment provides a culture of 3-hydroxypropionic acid producing cells having a high 3-hydroxypropionic acid content and a low by-product content. Specifically, it provides a culture of 3-hydroxypropionic acid producing cells, characterized by being prepared by the method for preparation of 3-HP and/or method for improvement of productivity of 3-HP.

The culture may have 3-hydroxypropionic acid of 60g/L or more, 62g/L or more, 65g/L or more, 67g/L or more, 70g/L or more, 72g/L or more, 75g/L or more, 77g/L or more, 78g/L, 79g/L or more, 80g/L or more, 81g/L or more, 82g/L or more, 83g/L or more, 84g/L or more, 85g/L or more, 86g/L or more, 87g/L or more, 88g/L or more, 89g/L or more, or 90g/L or more, based on the total culture (the upper limit may be selected from 85 to 1000g/L without special limitation, and for example, it may be 1000g/L, 500g/L, or 200g/L, but not limited thereto).

The culture may comprise at least one selected from the group consisting of acetic acid, orotic acid, propionic acid, succinic acid, formic acid, uracil acid and citric acid, and may comprise acetic acid at a content of 0 to 1 g/L, 0 to 0.5 g/L, 0 to 0.3 g/L, 0.01 to 1 g/L, 0.01 to 0.5 g/L or 0.01 to 0.3 g/L, for example, 0.3 g/L, based on the total culture having a high content of 3-hydroxypropionic acid (3-HP), but not limited thereto.

The culture may comprise orotic acid at a content of 0 to 0.5 g/L, 0 to 0.3 g/L, 0 to 0.2 g/L, 0 to 0.15 g/L, 0.01 to 0.5 g/L, 0.01 to 0.3 g/L, 0.01 to 0.2 g/L, or 0.01 to 0.15 g/L, for example, 0.14 g/L, based on the total culture having a high content of 3-hydroxypropionic acid (3-HP), but not limited thereto.

The culture may comprise propionic acid at a content of 0 to 0.5 g/L, 0 to 0.3 g/L, 0 to 0.2 g/L, 0.01 to 0.5 g/L, 0.01 to 0.3 g/L, or 0.01 to 0.2 g/L, for example, 0.19 g/L, based on the total culture having a high content of 3-hydroxypropionic acid (3-HP), but not limited thereto.

The culture may comprise succinic acid at a content of 0 to 1 g/L, 0 to 0.5 g/L, 0 to 0.3 g/L, 0 to 0.25 g/L, 0.01 to 1 g/L, 0.01 to 0.5 g/L, 0.01 to 0.3 g/L, or 0 to 0.3 g/L, for example, 0.2 g/L, based on the total culture having a high content of 3-hydroxypropionic acid (3-HP), but not limited thereto.

The culture may comprise formic acid at a content of 0 to 0.5 g/L, 0 to 0.3 g/L, 0 to 0.2 g/L, 0 to 0.15 g/L, 0.01 to 0.5 g/L, 0.01 to 0.3 g/L, 0.01 to 0.2 g/L, or 0.01 to 0.15 g/L, for example, 0.14 g/L, based on the total culture having a high content of 3-hydroxypropionic acid (3-HP), but not limited thereto.

The culture may comprise uracil acid at a content of 0 to 0.1 g/L, 0 to 0.05 g/L, 0 to 0.03 g/L, 0.01 to 0.1 g/L, 0.01 to 0.05 g/L, or 0.01 to 0.03 g/L, for example, 0.02 g/L, based on the total culture having a high content of 3-hydroxypropionic acid (3-HP), but not limited thereto.

The culture may comprise citric acid at a content of 0 to 0.1 g/L, 0 to 0.05 g/L, 0 to 0.02 g/L, or 0 to 0.01 g/L, based on the total culture having a high content of 3-hydroxypropionic acid (3-HP), but not limited thereto.

In one specific embodiment, the culture may be obtained by the method for preparation of 3-HP and/or method for improvement of productivity of 3-HP described above, and in the aspect of 3-HP preparation and productivity improvement, it may be advantageous to comprise by-products in the above content range.

In particular, in the culture, by-products may be comprised in the total culture having a high content of 3-HP produced during 3-HP production, and "orotic acid" is an intermediate of the pyrimidine biosynthesis pathway of a microorganism, and may be generated when 3-HP is produced by an aerobic microorganism.

The culture may comprise the by-products (for example, orotic acid), but it may be characterized by a low content of by-products, and for example, the culture produced by the method for preparation of 3-HP and/or method for improvement of productivity of 3-HP provided in the present description and/or the culture having a high content of 3-HP may have a lower content of by-products (for example, orotic acid), compared to a culture produced by a method for preparing 3-HP and/or a method for improving productivity of 3-HP in a different manner and/or a culture comprising 3-HP of different compositions.

The culture may be used for a use in producing 3-hydroxypropionic acid.

Accordingly, other embodiment provides a composition for producing 3-hydroxypropionic acid comprising the culture.

Other embodiment provides a method for producing 3-hydroxypropionic acid, comprising separating, recovering, and/or purifying 3-hydroxypropionic acid from the composition for producing 3-hydroxypropionic acid.

In another aspect, the present invention provides a high concentration culture method of cells having 3-hydroxypropionic acid productivity, comprising producing cells having 3-hydroxypropionic acid (3-HP) productivity at a concentration of $OD_{600}$ 5 to 23, 5 to 22, 5 to 17, 5 to 16, 5 to 10, 5 to 8, 6 to 23, 6 to 22, 6 to 17, 6 to 16, 6 to 10, 6 to 8, 10 to 23, 10 to 22, 10 to 17, 10 to 16, 14 to 23, 14 to 22, 14 to 17, 14 to 16, 17 to 23, 17 to 22, 20 to 23, or 20 to 22, for example, 7, 15, or 21 per hour.

[ADVANTAGEOUS EFFECTS]

The method for production and/or method for improving productivity of 3-HP provided in the present invention can produce 3-HP at a high concentration and high-productivity by applying a method for controlling the cell production rate by adjusting the sugar supply rate under a high-concentration cell culture condition, and a method for setting the conversion condition in the 3-HP production step. In this way, 3-HP is produced at a high concentration and high productivity, and thus, investment costs can be reduced compared to the same production amount, and an effect of reducing not only raw material costs but also separation and purification operating costs can be expected.

[BRIEF DESCRIPTION OF THE DRAWINGS]

FIG. 1 is a graph of the results measuring the production amount of 3-HP during 2-step culturing under the conditions of Example 1 and Example 2.

FIGs. 2a and 2b are graphs of the results measuring the production amount (g/L) of 3-HP during 2-step culturing under the conditions of Example 2-1 and Example 2-2, respectively.

FIGs. 3a and 3b are graphs of the results measuring the production amount (g/L) of 3-HP depending on the temperature of the culture solution during 3-HP production.

FIGs. 4a and 4b are graphs of the results measuring the production amount (g/L) of 3-HP depending on the pH of the culture solution during 3-HP production.

FIG. 5 is a graph of the results adjusting the temperature and pH conditions of the culture solution to (i) pH 7.25 and 37°C, (ii) pH 7.25 and 35°C, (iii) pH 6.85 and 37°C, and (iv) pH 6.8 and 35°C during 3-HP production, and in accordance with this, measuring the production amount (g/L) of 3-HP.

[MODE FOR INVENTION]

Hereinafter, the present invention will be described by examples in more detail. However, the following examples are intended to illustrate the contents of the present invention only, but the scope of the present invention is not limited by the following examples.

In the present invention, unless otherwise mentioned, all temperatures described herein are in degrees Celsius.

Reference Example 1. Preparation of 3-HP producing strain

A 3-HP producing strain to be used for the method for preparation according to the present invention, was prepared according to the method disclosed in Korean Patent Application No. 10-2021-0151641, and was used for the following examples.

Example 1. Production of 3-HP by applying adaptation conditions and optimal conditions (2-step culturing + controlling $OD_{600}$ value)

Example 1-1. High-concentration culture of cells (first step culturing)

The 3-HP producing strain prepared in Reference Example 1 was under high-concentration cell culture with a 5L fermenter (Working volume 2L) by a fed-batch culture method.

Specifically, 20 g/L of glucose was separately added to an MR medium ($KH_2PO_4$ 6.67g, $(NH_4)_2HPO_4$ 4g, $MgSO_4 \cdot 7H_2O$ 0.8g, citric acid 0.8g, and trace metal solution 5mL per 1L; herein, Trace metal solution is 5M HCl 5mL, $FeSO_4 \cdot 7H_2O$ 10g, $CaCl_2$ 2g, $ZnSO_4 \cdot 7H_2O$ 2.2g, $MnSO_4 \cdot 4H_2O$ 0.5g, $CuSO_4 \cdot 5H_2O$ 1g, $(NH_4)_6Mo_7O_2 \cdot 4H_2O$ 0.1g, and $Na_2B_4O_2 \cdot 10H_2O$ 0.02g per 1L), and it was used as a cell culture medium, and the temperature of 35°C was maintained. The pH was maintained at 6.95 using ammonia water.

For the high-concentration cell culture, a fed-batch culture, specifically, a pH-stat feeding method or DO-stat feeding method, and a glucose solution at a concentration of 700g/L per 1L of the medium was supplied at 17.5 mL/h on average at the time when the glucose concentration in the culture medium became 0 g/L so that sugars which were added in the culture medium were consumed all by the microorganisms and the glucose concentration could be maintained at 0 g/L, and the optical density (OD) was measured using a UV-spectrometer to measure the cell concentration, and the $OD_{600}$ was controlled to range from 6 to 21. At 24 hours after starting the culturing, $OD_{600}$ was measured at about 150 (dry cell weight 50g/L).

After completing the high-concentration cell culture, the cell culture solution was used as it was without a separate recovery process in the subsequent step.

Example 1-2. 3-HP production using high-concentration cultured cells (second step culturing)

A medium for producing 3-HP was prepared by adding 190g/L of glycerol and 10$\mu$M vitamin B12 to 10mM phosphate buffer comprising no glucose. The cell culture solution prepared in Example 1-1 was inoculated to the medium for producing 3-HP so that the cell inoculation amount was 10g/L (based on dry cell weight), and a 3-HP producing step was performed in a 5L fermenter (Working volume 2L).

As 3-HP production conditions, DO 5% was maintained, and the stirring speed of the culture solution was performed at 300 rpm so that the DO change value was less than 2, and aeration was sequentially adjusted by supplying at 1 vvm to form an aerobic condition. Other culture conditions were maintained at the temperature of 37°C and the pH of 7.0 using $Ca(OH)_2$.

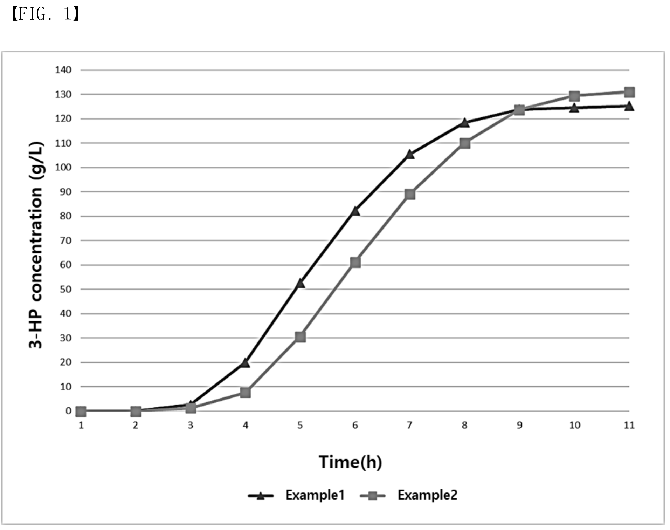

The production amount (production concentration) (g/L) of 3-HP produced in the corresponding process was measured by HPLC analysis, and the results were shown in FIG. 1 (Example 1).

Example 2. Air supply control conditions for electron transport system (2-step culturing + controlling DO)

Example 2-1. High-concentration culture of cells (first step culturing)

High-concentration cell culture was performed by the substantially same method as Example 1-1, and after completing the high-concentration cell culture, the cell culture solution was used as it was without a separate recovery process in the subsequent step.

Example 2-2. 3-HP production using high-concentration cultured cells (second step culturing)

In order to produce high concentrations of 3-HP under stable rather than optimal conditions, thereby overcoming production inhibition, the following method was used.

Specifically, a medium for producing 3-HP was prepared by adding 190g/L of glycerol and 10$\mu$M vitamin B12 to 10mM phosphate buffer comprising no glucose. The cell culture solution prepared in Example 2-1 was inoculated to the medium for producing 3-HP so that the cell inoculation amount was 10g/L (based on dry cell weight), and a 3-HP producing step was performed in a 5L fermenter (Working volume 2L).

As 3-HP production conditions, DO 5% was maintained, and the stirring speed of the culture solution was performed at 300 rpm so that the DO change value was less than 2, and aeration was sequentially adjusted by supplying at 1 vvm to form an aerobic condition.

In addition, the experiment included a step of applying temperature and pH conditions that considered cell stability, instead of the initial production conditions, to stabilize the cells. It also included a step in which long-term stability conditions were applied after the adaptation and initial optimal conditions during the 3-HP production process.

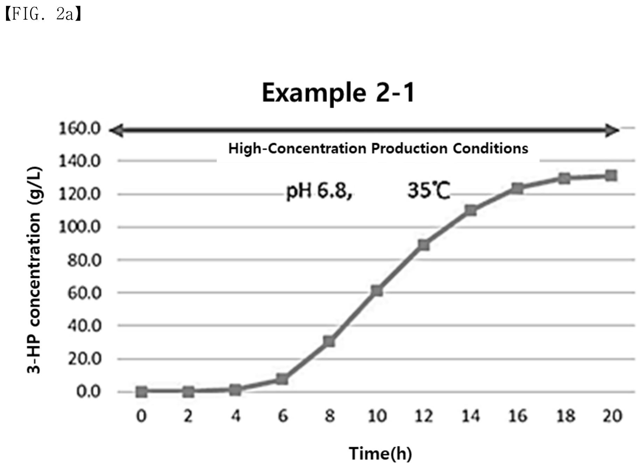

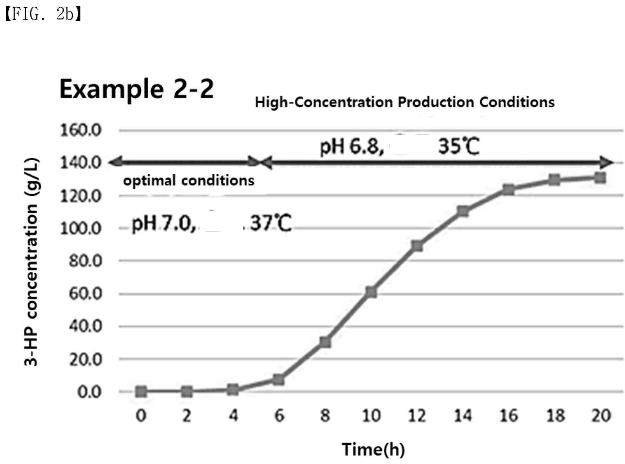

Specifically, the step of producing 3-HP was performed by maintaining the pH 6.8 and 35°C temperature conditions to produce high-concentration 3-HP by maintaining the high-concentration production conditions from the beginning (Example 2-1), and in order to produce high-concentration 3-HP by starting with the optimal conditions at the beginning and converting into the high-concentration production conditions from the middle stage, for about 5 hours from the beginning of fermentation, the conditions of pH 7.0 and 37°C were maintained, and after that, the high-concentration production conditions of pH 6.8 and 35°C were maintained to produce 3-HP (Example 2-2), and the 3-HP production amount (production concentration) (g/L) produced according to each process was measured by HPLC analysis, and the measurement results of each process were shown in FIG. 2a and FIG. 2b. In addition, based on the measured 3-HP production amount, the 3-HP production yield by Equation 1 below, and the 3-HP productivity by Equation 2 below were measured:

3-HP production yield (%) = [(Final 3-HP(g))/{( Glycerol before culturing (g)) - (Glycerol remaining after culturing (g))}] * 100       [Equation 1]

3-HP productivity = (Final 3-HP production concentration (g/L))/(Total 3-HP production time)       [Equation 2]

As a result, both Example 2-1 and Example 2-2 showed consistent outcomes: a 3-HP production concentration of 131 g/L, a production yield of 95%, and a productivity of 6.55 g/L/h. Since the production concentration profiles of both methods were substantially the same, the results were expressed at once as Example 2 and are shown in FIG. 1. Additionally, the composition of the culture containing 3-hydroxypropionic acid, cultivated under conditions of pH 6.8 and 35° C, was analyzed using HPLC, and the results are summarized in Table 1 below.

[Table 1]

| Composition | Concentration (g/L) |
| --- | --- |
| 3-hydroxypropionic acid | 131.1 |
| Glycerol | 0.1 |
| Acetic acid | 0.3 |
| Orotic acid | 0.14 |
| Propionic acid | 0.19 |
| Succinic acid | 0.2 |
| Formic acid | 0.1 |
| Uracil acid | 0.02 |
| Citric acid | - |

Example 3. Comparison of 3-HP production amount depending on culture temperature during 3-HP production

The production amount (g/L) of 3-HP depending on the culture temperature during 3-HP production was measured while producing 3-HP by the method of Example 1.

Specifically, after the first step culturing by the method of Example 1-1, the culture solution comprising the strain for producing 3-HP (strain prepared in Reference Example 1), was inoculated under the same conditions as the second step culturing of Example 1-2 to perform culturing, and the 3-HP production amount was measured by the substantially same method.

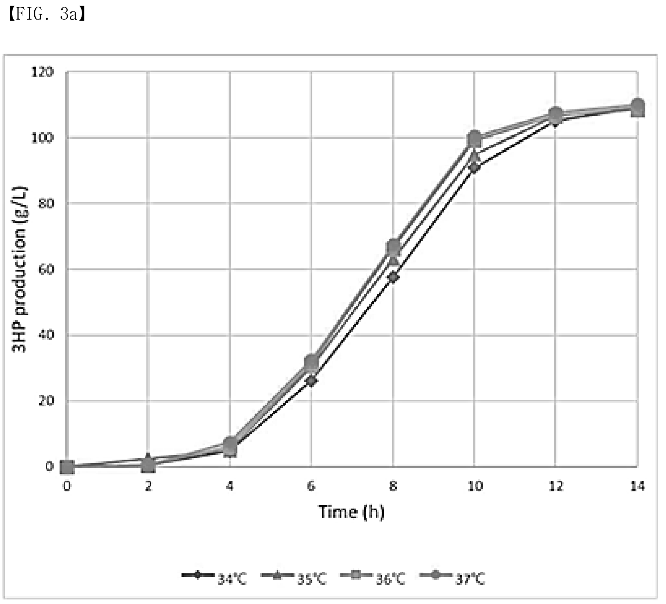

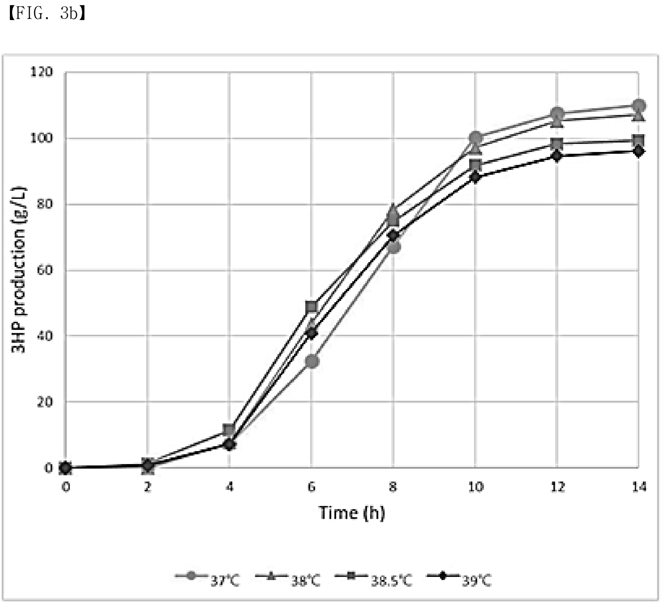

However, the culture temperature in the second step culturing was adjusted to 34, 35, 36 and 37, 38, 38.5 and 39°C, respectively, and the results of measuring the 3-HP production amount were shown in FIG. 3a and FIG. 3b.

As shown in FIG. 3a, as the culture temperature decreased below 37°C, the production rate decreased, but the enzyme stability of the cells was maintained, so all glycerol was consumed and 3-HP was produced at the same yield.

In addition, as shown in FIG. 3b, as the temperature was higher based on the culture temperature 37°C, the initial productivity was better, but the stability of the cells was reduced, and therefore, the production concentration of 3-HP was lowered due to cell activity degradation.

Example 4. Comparison of 3-HP production amount depending on culture pH during 3-HP production

The production amount (g/L) of 3-HP depending on the pH of the culture solution during 3-HP production was measured while producing 3-HP by the method of Example 1.

Specifically, after the first step culturing by the method of Example 1-1, the culture solution comprising the strain for producing 3-HP (strain prepared in Reference Example 1), was inoculated under the same conditions as the second step culturing of Example 1-2 to perform culturing, and the 3-HP production amount was measured by the substantially same method.

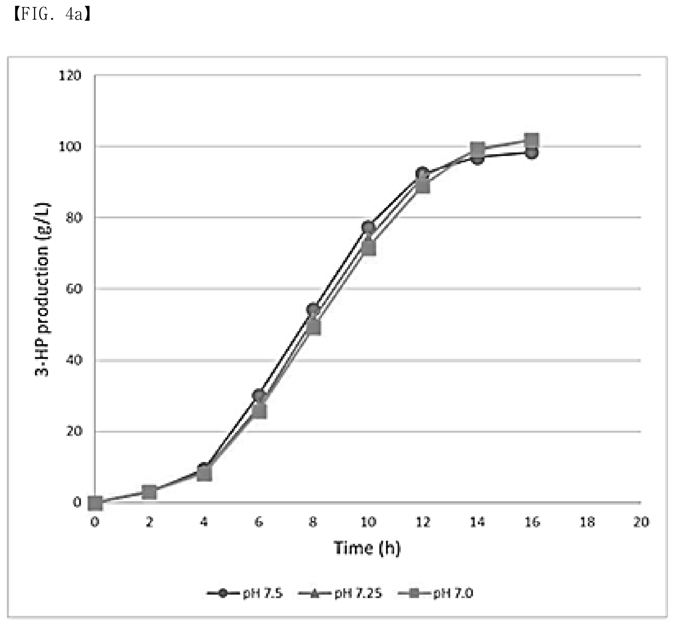

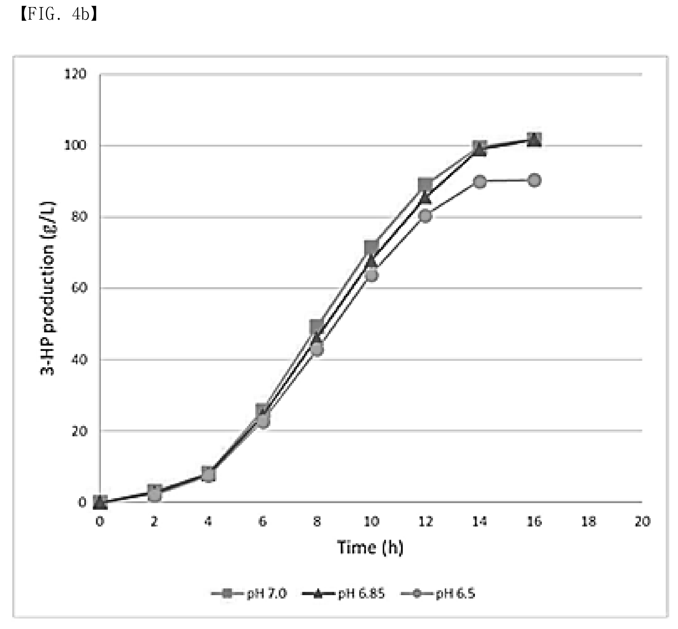

However, the culture pH in the second step culturing was adjusted to 6.5, 6.85, 7.0, 7.25 and 7.5, respectively, and the results of measuring the 3-HP production amount were shown in FIG. 4a and FIG. 4b.

As shown in FIG. 4a, the good initial productivity was shown when the culture pH was 7.5, but due to cell activity degradation, as the culture time was beyond 10 hours, the cell stability was reduced.

In addition, as shown in FIG. 4b, at pH 6.5, the cell stability was reduced in the later stage of culture and 3-HP production stopped, but at pH 6.85, the initial productivity was slightly slower than 7.0, but constant productivity was shown, so the cell stability was demonstrated. In other words, the stable pH conditions were shown as 6.85 to 7.25.

Example 5. Comparison of 3-HP production amount depending on cell production per hour in high-concentration cell culturing step during 3-HP production process

The production amount (g/L) of 3-HP depending on the cell production amount per hour during high-concentration cell culture was measured while performing high-concentration cell culture and producing 3-HP by the method of Example 1.

In the first step culturing by the method of Example 1-1, as the cell production speed, the cell production amount per hour was determined by the supply rate of the glucose solution added to the culture medium, the high-concentration cell culture solutions with different cell production per hour by varying the glucose supply rate were inoculated under the same conditions as the second step culturing of Example 1-2 to perform culturing, and the cell concentration (cell production amount) and 3-HP production amount (g/L) per hour were measured by the substantially same method as Example 1-1 and 1-2.

Specifically, the first step culturing was performed by adjusting the glucose supply rate to 5, 10, 20, 30 or 40 mL/L/h, and the pH and temperature conditions in the second step culturing were set to pH 6.8 and 35°C, respectively, and the experiment was conducted in the 145 g/L glycerol medium, and the results of measuring the 3-HP production amount were shown in Table 2.

[Table 2]

| Glucose supply rate (mL/L/h) | Cell production amount per hours ($OD_{600}$/h) | 3-HP production concentration (g/L) |
|---|---|---|
| 5 | 4 | 62 |
| 10 | 7 | 104 |
| 20 | 15 | 109 |
| 30 | 21 | 101 |
| 40 | 25 | 83 |

As a result of measuring the 3-HP production amount, in the first step culturing, under the condition in which the glucose supply rate was 20, that is, when the cell production amount per hour was $OD_{600}$ 15, the 3-HP production concentration was most excellent.

**Example 6. High-concentration 3-HP production condition search**

In order to produce a higher concentration of 3-HP than the condition producing the highest concentration of 3-HP under the optimal conditions (temperature, pH, glycerol concentration) searched in Examples 3 and 4, considering the cell stability, the effect of the conditions of 35°C and pH 6.85, which showed the same production, although the initial production rate was slightly slow, on 3-HP production was evaluated.

Specifically, after the first step culturing by the method of Example 1-1, the culture solution comprising the strain for producing 3-HP (strain prepared in Reference Example 1), was inoculated under the same conditions as the second step culturing of Example 1-2 to perform culturing, and the 3-HP production amount was measured by the substantially same method.

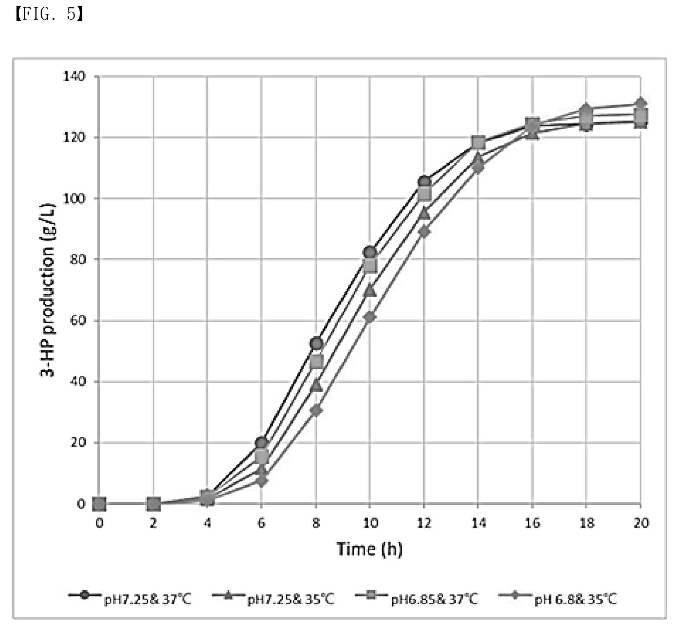

However, the culture pH and temperature conditions were adjusted to (i) pH 7.25 and 37°C, (ii) pH 7.25 and 35°C, (iii) pH 6.85 and 37°C, and (iv) pH 6.8 and 35°C, respectively. To achieve high-concentration 3-HP production, the experiment was conducted in a glycerol medium of 190 g/L. The 3-HP production results for each condition are shown in FIG. 5.

As shown in FIG. 5, when comparing the conditions where the pH was maintained at 7.25 and only the culture temperature was varied between 37°C and 35°C, the production of 3-HP at 35°C was 125 g/L, which was the same as at 37°C.

When comparing the cases where the culture temperature was maintained at 37°C, and only the pH was adjusted to 7.25 and 6.85, respectively, in the case where the pH was lowered to 6.85, 3-HP of 127.5 g/L, higher than the 3-HP production amount in case of pH 7.25 (125g/L) was produced.

In addition, when comparing the 3-HP production amount when all the culture temperature and pH condition were different, the 3-HP production amount under the conditions of the culture temperature 37C and pH 7.25 was 125 g/L, while the 3-HP production amount under the conditions of the culture temperature 35°C and pH 6.85 where both temperature and pH were low, was 131 g/L.

**Claims**

1. A method for producing 3-hydroxypropionic acid comprising the following steps:

   (1) high-concentration cell culturing which conducts cell culture of cells having productivity of 3-hydroxypropionic acid in a culture medium until the cell concentration reaches an $OD_{600}$ of 100 to 200; and
   (2) producing 3-hydroxypropionic acid while maintaining the state in which dissolved oxygen is 1 to 20% by transferring the culture solution of the step (1) to a production medium comprising a substrate,

   wherein the step (2) is performed under the following conditions:

   (i) the temperature condition of 30 to 40°C; and/or
   (ii) the pH condition of 6 to 8.

2. The method according to claim 1, wherein the cells having productivity of 3-hydroxypropionic acid comprises a gene encoding at least one protein selected from the group consisting of glycerol dehydratase and aldehyde dehydrogenase.

3. The method according to claim 1, wherein the culture medium of the step (1) does not comprise glycerol as a carbon source.

4. The method according to claim 3, wherein the carbon source is glucose.

5. The method according to claim 1, wherein a carbon source at a content of 4 to 25 g/L per hour based on the volume of the culture medium is further supplied to the culture medium in the step (1), and g/L is a concentration in weight of carbon source added g/ volume of medium L.

6. The method according to claim 1, wherein the culture solution itself is transferred to the production medium without passing through an additional step including cell recovery, after the culturing of the step (1).

7. The method according to claim 1, wherein the change value of the dissolved oxygen of the step (2) is 0 to 5%.

8. The method according to claim 1, wherein the maintaining the dissolved oxygen in the step (2) is performed by adjusting the stirring speed and air supply.

9. The method according to claim 1, wherein the step (2)

(i) is performed under the temperature condition of 37°C to 40°C or the pH condition of 7 to 8, and
(ii) is performed under the temperature condition of 30°C or more to less than 37°C or the pH condition of 6 or more to less than 7.

10. The method for producing 3-hydroxypropionic acid according to any one claim of claim 1 to claim 9, having productivity of 3-hydroxypropionic acid of 6 g/L/h or more.

11. A culture of a cell producing 3-hydroxypropionic acid, which is prepared by the method for producing 3-hydroxypropionic acid according to any one claim of claim 1 to claim 9.

12. A composition for preparing 3-hydroxypropionic acid, comprising the culture of claim 11.

13. A culture of a cell producing 3-hydroxypropionic acid, wherein the culture is 3-hydroxypropionic acid and its by-product, and comprises orotic acid of 0.01 to 0.5 g/L.

14. The culture of a cell producing 3-hydroxypropionic acid according to claim 13, wherein the culture of a cell producing 3-hydroxypropionic acid further comprises at least one by-product selected from the group consisting of acetic acid of 0.01 to 1 g/L, propionic acid of 0.01 to 0.5 g/L, succinic acid of 0.01 to 1 g/L, formic acid of 0.01 to 0.5 g/L, uracil acid of 0.01 to 0.1 g/L, and citric acid of 0.01 to 0.1 g/L as a by-product.

15. A method for high-concentration culturing of a cell having productivity of 3-hydroxypropionic acid, comprising producing a cell having productivity of 3-hydroxypropionic acid at a concentration of $OD_{600}$ 5 to 23 per hour.

【FIG. 1】

【FIG. 2a】

Example 2-1

【FIG. 2b】

Example 2-2

High-Concentration Production Conditions

【FIG. 3a】

【FIG. 3b】

【FIG. 4a】

【FIG. 4b】

【FIG. 5】

## INTERNATIONAL SEARCH REPORT

| International application No. |
| --- |
| **PCT/KR2025/001247** |

| A. | CLASSIFICATION OF SUBJECT MATTER |
| --- | --- |

**C12P 7/52**(2006.01)i; **C12P 7/42**(2006.01)i; **C12N 9/88**(2006.01)i; **C12N 9/02**(2006.01)i

According to International Patent Classification (IPC) or to both national classification and IPC

| B. | FIELDS SEARCHED |
| --- | --- |

Minimum documentation searched (classification system followed by classification symbols)

C12P 7/52(2006.01); C12N 1/20(2006.01); C12N 15/70(2006.01); C12P 7/42(2006.01); C12Q 1/26(2006.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산(3-hydroxypropionic acid; 3-HP), 고농도 세포 배양(high density cell culture), 용존산소량(dissolved oxygen; DO), 오로트산(orotic acid)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| DX | KR 10-2022-0061044 A (LG CHEM, LTD.) 12 May 2022 (2022-05-12)<br>See paragraphs [0047], [0063]-[0064], [0072], [0102], [0109]-[0119] and [0126]-[0128]; and claims 1, 6 and 9-12. | 1-12,15 |
| A | | 13-14 |
| X | KR 10-2022-0015996 A (LG CHEM, LTD.) 08 February 2022 (2022-02-08)<br>See paragraphs [0033] and [0087]-[0091]; claim 1; and figure 1. | 15 |
| A | US 2015-0044746 A1 (OPX BIOTECHNOLOGIES, INC.) 12 February 2015 (2015-02-12)<br>See entire document. | 1-15 |
| A | TOKUYAMA, Kento et al. Increased 3-hydroxypropionic acid production from glycerol, by modification of central metabolism in Escherichia coli. Microbial Cell Factories. 07 May 2014, vol. 13, 64, pp. 1-11.<br>See entire document. | 1-15 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **21 April 2025** | **21 April 2025** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2025/001247** |

**C.    DOCUMENTS CONSIDERED TO BE RELEVANT**

| Category* | Citation of document, with indication, where appropriate, of the relevant passages | Relevant to claim No. |
|---|---|---|
| A | ASHOK, Somasundar et al. Production of 3-Hydroxypropionic Acid From Glycerol by Recombinant Klebsiella pneumoniae △dhaT△yqhD Which Can Produce Vitamin B12 Naturally. Biotechnology and Bioengineering. 05 October 2012 (Online), vol. 110, no. 2, pp. 511-524.<br>See entire document. | 1-15 |
| A | KR 10-2014-0015998 A (SAMSUNG ELECTRONICS CO., LTD. et al.) 07 February 2014 (2014-02-07)<br>See entire document. | 1-15 |

Form PCT/ISA/210 (second sheet) (July 2022)

**INTERNATIONAL SEARCH REPORT**
Information on patent family members

International application No.

**PCT/KR2025/001247**

| Patent document cited in search report | | | Publication date (day/month/year) | Patent family member(s) | | | Publication date (day/month/year) |
|---|---|---|---|---|---|---|---|
| KR | 10-2022-0061044 | A | 12 May 2022 | CN | 116323957 | A | 23 June 2023 |
| | | | | EP | 4242319 | A1 | 13 September 2023 |
| | | | | EP | 4242319 | A4 | 22 May 2024 |
| | | | | KR | 10-2696014 | B1 | 16 August 2024 |
| | | | | US | 2023-0340547 | A1 | 26 October 2023 |
| | | | | WO | 2022-098162 | A1 | 12 May 2022 |
| KR | 10-2022-0015996 | A | 08 February 2022 | CN | 115956123 | A | 11 April 2023 |
| | | | | EP | 4190907 | A1 | 07 June 2023 |
| | | | | EP | 4190907 | A4 | 06 December 2023 |
| | | | | JP | 2023-531938 | A | 26 July 2023 |
| | | | | JP | 2024-163267 | A | 21 November 2024 |
| | | | | KR | 10-2639658 | B1 | 22 February 2024 |
| | | | | US | 2023-0265466 | A1 | 24 August 2023 |
| | | | | WO | 2022-025710 | A1 | 03 February 2022 |
| US | 2015-0044746 | A1 | 12 February 2015 | US | 2016-0130614 | A1 | 12 May 2016 |
| | | | | WO | 2014-145297 | A1 | 18 September 2014 |
| KR | 10-2014-0015998 | A | 07 February 2014 | US | 2014-0093901 | A1 | 03 April 2014 |

Form PCT/ISA/210 (patent family annex) (July 2022)

**EP 4 692 362 A1**

**Patent documents cited in the description**

- KR 1020240009485

- KR 1020210151641